Europäisches Patentamt

European Patent Office

Office européen des brevets

(11) Veröffentlichungsnummer: **0 054 690**
**B1**

(12) # EUROPÄISCHE PATENTSCHRIFT

(45) Veröffentlichungstag der Patentschrift:
29.01.86

(51) Int. Cl.⁴: **A 61 K 9/00**, A 61 K 33/26

(21) Anmeldenummer: 81108947.3

(22) Anmeldetag: 27.10.81

(54) **Natriumnitroprussid-Natriumthiosulfat-Perfusor-Kit.**

(30) Priorität: 20.12.80 DE 3048211

(43) Veröffentlichungstag der Anmeldung:
30.06.82 Patentblatt 82/26

(45) Bekanntmachung des Hinweises auf die Patenterteilung:
29.01.86 Patentblatt 86/5

(84) Benannte Vertragsstaaten:
AT BE CH DE FR GB IT LI LU NL SE

(56) Entgegenhaltungen:
US - A - 2 176 041

CHEMICAL ABSTRACTS, Band 94, Nr. 17, 27. April 1981, Seite 180, Nr. 133355g, Columbus, Ohio, USA, M. HOEBEL et al.: "The antidote effect of thiosulfate and hydroxocobalamin in formation of nitroprusside intoxication of rabbits"
CHEMICAL ABSTRACTS, Band 90, Nr. 13, 26. März 1979, Nr. 97633c, Columbus, Ohio, USA, M. HOEBEL et al.: "Effect of sodium nitroprusside alone and in combination with sodium thiosulfate on the acid base balance, and on thiocyanate and iron plasma levels in the rabbit"
CHEMICAL ABSTRACTS, Band 87, Nr. 13, 26. September 1977, Seite 95, Nr. 96048s, Columbus, Ohio, USA, P. BOUYARD et al.: "Toxico-pharmacological study of

(73) Patentinhaber: **Schulz, Dr. Volker, Laudahnstrasse 37, D-5000 Köln 41 (DE)**

(72) Erfinder: **Schulz, Dr. Volker, Laudahnstrasse 37, D-5000 Köln 41 (DE)**

(74) Vertreter: **von Kreisler, Alek, Dipl.-Chem. et al, Deichmannhaus am Hauptbahnhof, D-5000 Köln 1 (DE)**

(56) Entgegenhaltungen: (Fortsetzung)
lyophilized sodium nitroprusside"
CHEMICAL ABSTRACTS, Band 92, Nr. 19, 12. Mai 1980, Seite 349, Nr. 169260v, Columbus, Ohio, USA
Intensivmed: Bd. 16, p. 320-325 (1979)

ACTORUM AG

## Beschreibung

Natriumnitroprussid ist seit 1975 als Fertigarzneimittel zur Anwendung am Menschen im Handel (Nipruss[1] Pharma Schwarz und Nipride[1] Hoffmann-LaRoche). Die beiden Arzneimittelspezialitäten bestehen aus Trockenampullen, die 50 mg bzw. 60 mg Lyophilisat enthalten, das in bestimmten handelsüblichen Infusionslösungen zu lösen ist. In gelöster Form wird es dann als intravenöse Dauerinfusion gegeben. Die Blutdrucksenkung setzt zu Beginn der Infusion innerhalb weniger Minuten ein und klingt bei Unterbrechung der Infusion ebenso schnell wieder ab. Die Senkung des Druckes ist dem Dosisstrom proportional.

Wegen seiner ausserordentlich starken Wirkung und guten Steuerbarkeit ist Natriumnitroprussid insbesondere für bestimmte Notfallbehandlungen ein unverzichtbares Pharmakon und wurde als solches 1979 auch in die WHO-Liste der unentbehrlichen Medikamente aufgenommen.

Natriumnitroprussid besteht zu 44 Gew.-% aus Zyanidionen. Die Zyanidionen werden im Blut freigesetzt und können bei entsprechender Dosierung und Infusionsdauer zu Zyanidvergiftungen führen. In den letzten Jahren wurden mehrere tödliche Vergiftungsfälle bekannt, die durch die Nitroprussid-Therapie verursacht wurden.

Eine multizentrische klinische Studie, die gegenwärtig vom Anmelder organisiert und geleitet wird, hat ergeben, dass selbst bei kurzfristigen Anwendungen von Nitroprussid bei etwa 20% der Behandelten mit gefährlichen Zyanidkumulationen zu rechnen ist.

Tierversuche ergaben, dass sich die Zyanidentgiftung im Körper mit Hilfe von Thiosulfat-Infusionen stark beschleunigen lässt.

Der Anmelder hat in geeigneten Untersuchungen nachgewiesen, dass die forcierte Zyanidentgiftung mit Thiosulfat auch bei Patienten während der Nitroprussid-Therapie wirksam ist. Die dafür erforderliche Thiosulfatdosis hat für den betreffenden Patienten keinerlei schädigende Nebenwirkungen. Das Thiosulfat muss jedoch wegen seiner kurzen biologischen Halbwertzeit als Dauerinfusion simultan mit dem Natriumnitroprussid infundiert werden. Ausserdem müssen die Dosisströme von Nitroprussid und Thiosulfat äquivalent aufeinander abgestimmt sein.

Eine Zusammenfassung findet sich in «Intensivmed» 16 (1979) 320–325, in «Nieren- und Hochdruckkrankheiten» Jahrg. 9, Nr. 6/1980 sowie in «Klinische Wochenschrift» 57 (1979) 905–907.

Die proportionale Infusion von Nitroprussid und Thiosulfat über zwei separate Infusionssysteme bereitet jedoch in der klinischen Praxis Schwierigkeiten, u.a. weil der Dosisstrom der Nitroprussidinfusion im Verlauf der Behandlung sehr häufig an wechselnde Blutdrucke angepasst werden muss.

In Intensivmed. Bd. 16, s. 320–325 (1979) werden die Mischungsverhältnisse von Nitroprussid/Thiosulfat und ein Applikationsverfahren beschrieben, bei dem prophylaktisch infundiertes Natriumthiosulfat und Nitroprussid in den gleichen Katheter eingeführt werden. Dabei handelt es sich um ein sog. Bolusverfahren, bei dem diskontinuierlich (abwechselnd) Nitroprussid und Thiosulfat zugegeben werden (s. J. of Cardiovasculat Pharmacology, S. 77–85 (1983)).

Diese Verfahren haben jedoch grosse praktische Nachteile, die insbesondere bei intensiv medizinischen Behandlungen zur Gefährdung der Patienten führen können. Daher besteht ein Bedarf nach einer unter den praktischen Gegebenheiten der Intensivmedizin zuverlässigen und für den Patienten gefahrlosen Lösung.

Die Infusion eines proportionierten Lösungsgemisches von Nitroprussid und Thiosulfat scheiterte andererseits daran, dass diese Mischlösung hochgradig lichtempfindlich ist, so dass sie mit dem herkömmlichen Infusionsverfahren nicht angewendet werden konnte.

Die Erfindung stellt sich die Aufgabe, diese Nachteile des Standes der Technik zu beseitigen.

Untersuchungen des Anmelders haben ergeben, dass eine wässrige Mischlösung von Nitroprussid und Thiosulfat bei totalem Lichtausschluss wochenlang ohne einen Verlust der pharmakologischen Wirkung bei Zimmertemperatur haltbar ist. Aufgrund geeigneter Untersuchungen zur Dosierung und unter Berücksichtigung der in den Kliniken zur Verfügung stehenden Infusionsgeräte hat der Anmelder die Wirksamkeit der folgenden Mischlösungen bei einer geeigneten Zahl von Patienten geprüft:

| | |
|---|---|
| Natriumthiosulfat | 500 mg |
| Natriumnitroprussid | 50 mg |
| Aqua Dest. | ad 50 ml |

Diese Mischlösung wurde in geeigneter Weise unter totalem Lichtausschluss zubereitet und bei entsprechender Indikation an Patienten infundiert. Im Vergleich mit gleichkonzentrierten wässrigen Lösungen von Natriumprussid ohne Thiosulfat war die blutdrucksenkende Wirkung der Mischlösung intensiver. Dieser potenzierte Effekt des Thiosulfates ist möglicherweise aufgrund der aus der Literatur bekannten Hemmung der Nitroprussidwirkung durch kumulierendes Zyanid zu erklären. Die Zyanidkonzentrationen im Blut stiegen bei den Patienten während der Infusion der Mischlösung auf weniger als $1/10$ derjenigen Spiegel an, die bei der Infusion äquivalenter Mengen von Nitroprussid als Monoinfusion gemessen wurden. Zyanidvergiftungen im Rahmen der Nitroprussid-Therapie sind infolgedessen bei der Infusion als Mischlösung mit Thiosulfat nicht mehr möglich.

Gegenstand der Erfindung ist dementsprechend ein Natriumnitroprussid-Thiosulfat-Perfusor-Ki zur Bluthochdrucksenkung durch Infusion einer Mischlösung, enthaltend Natriumthiosulfat und Natriumnitroprussid, dadurch gekennzeichnet, dass er folgende Bestandteile enthält:

1 Flasche mit Durchstechstopfen enthaltend Natriumthiosulfatlösung;

1 Perfusorspritze aus lichtundurchlässigem Kunststoff mit beiliegender Kanüle zum Aufziehen der Lösung;

1 Perfusorschlauch aus lichtundurchlässigem Kunststoff;

1 Trockenampulle, enthaltend lyophilisiertes Natriumnitroprussid.

Bei einer bevorzugten Ausführungsform enthält der erfindungsgemässe Kit die folgenden Bestandteile;

1 Flasche mit Durchstechstopfen, enthaltend 50 ml 1%ige Natriumthiosulfatlösung;

1 50 ml-Perfusorspritze aus lichtundurchlässigem Kunststoff mit beiliegender Kanüle zum Aufziehen der Lösung;

1 Perfusorschlauch aus lichtundurchlässigem Kunststoff;

1 Trockenampulle, enthaltend 50 mg lyophilisiertes Natriumnitroprussid.

Der erfindungsgemässe Kit wird wie folgt angewendet.

Die Thiosulfatlösung wird mit der beiliegenden Kanüle in die lichtundurchlässige Perfusorspritze aufgezogen. Der Inhalt der Nitroprussid-Ampulle wird mit einer kleinen Portion des Spritzeninhaltes gelöst, sofort wieder in die Spritze aufgezogen und darin umgeschüttelt. Der lichtundurchlässige Schlauch wird aufgeschraubt und in senkrechter Spritzenstellung wird die Luft aus dem System entfernt. Die Spritze wird auf den Perfusor aufgelegt, dieser einen kurzen Moment mit schnellem Vorschub eingestellt, bis das Schlauchende tropft. Dann wird das Schlauchende über ein T-Stück unmittelbar an den Venenkatheter im bypass an eine laufende Infusion an den Patienten angeschlossen. Für die Infusion, deren Flussgeschwindigkeit zur Vermeidung verzögerter Blutdruckeinstellung 20 ml/Std. oder mehr betragen soll, sind alle gebräuchlichen Infusionslösungen geeignet. Die Perfusoreinstellung beginnt mit 1 ml/Std. und wird nach Bedarf gesteigert.

Bei einer anderen Ausführungsform kann der erfindungsgemässe Natriumnitroprussid-Thiosulfat-Perfusor-Kit zur Bluthochdrucksenkung durch Infusion einer Mischlösung der obigen Verbindungen auch durch folgende Bestandteile gekennzeichnet sein:

1 lichtundurchlässige Flasche mit Durchstechstopfen, enthaltend eine Mischlösung von Natriumthiosulfatlösung und darin gelöstem Natriumnitroprussid;

1 Perfusorspritze aus lichtundurchlässigem Kunststoff mit beiliegender Kanüle zum Aufziehen der Lösung;

1 Perfusorschlauch aus lichtundurchlässigem Kunststoff.

Vorzugsweise ist bei dieser Ausführungsform der Kit durch folgende Bestandteile gekennzeichnet:

1 lichtundurchlässige Flasche mit Durchstechstopfen, enthaltend eine Mischlösung von 50 ml 1%ige Natriumthiosulfatlösung und 50 mg darin gelöstem Natriumnitroprussid;

1 50 ml-Perfusorspritze aus lichtundurchlässigem Kunststoff mit beiliegender Kanüle zum Aufziehen der Lösung;

1 Perfusorschlauch aus lichtundurchlässigem Kunststoff.

Die Erfindung wird anliegend anhand von Abbildungen erläutert, die graphische Darstellungen von klinischen Versuchen zeigen.

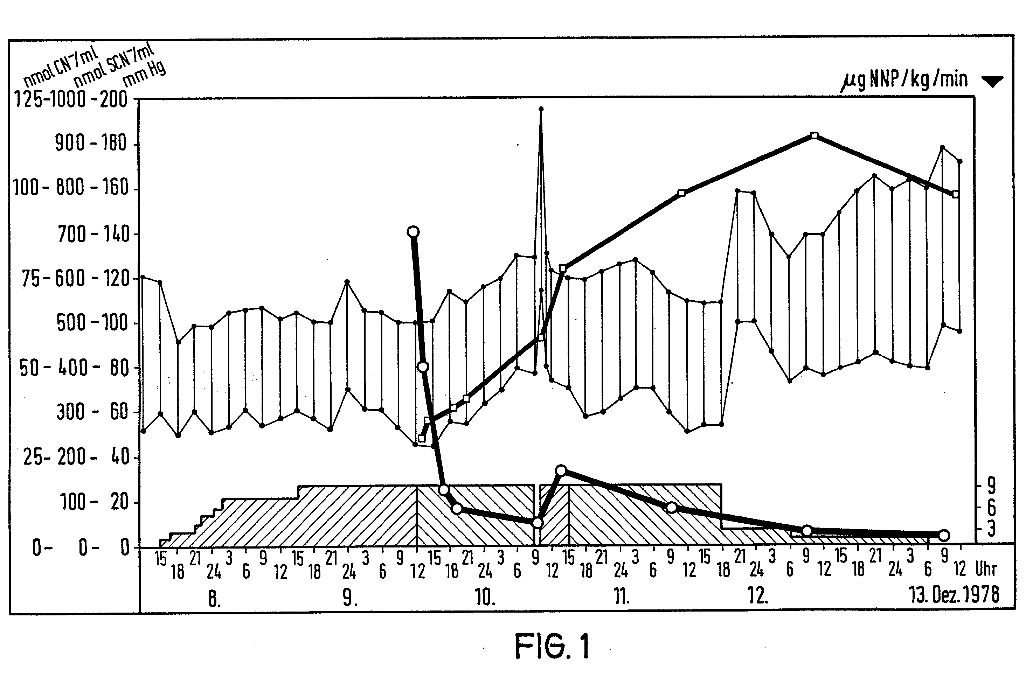

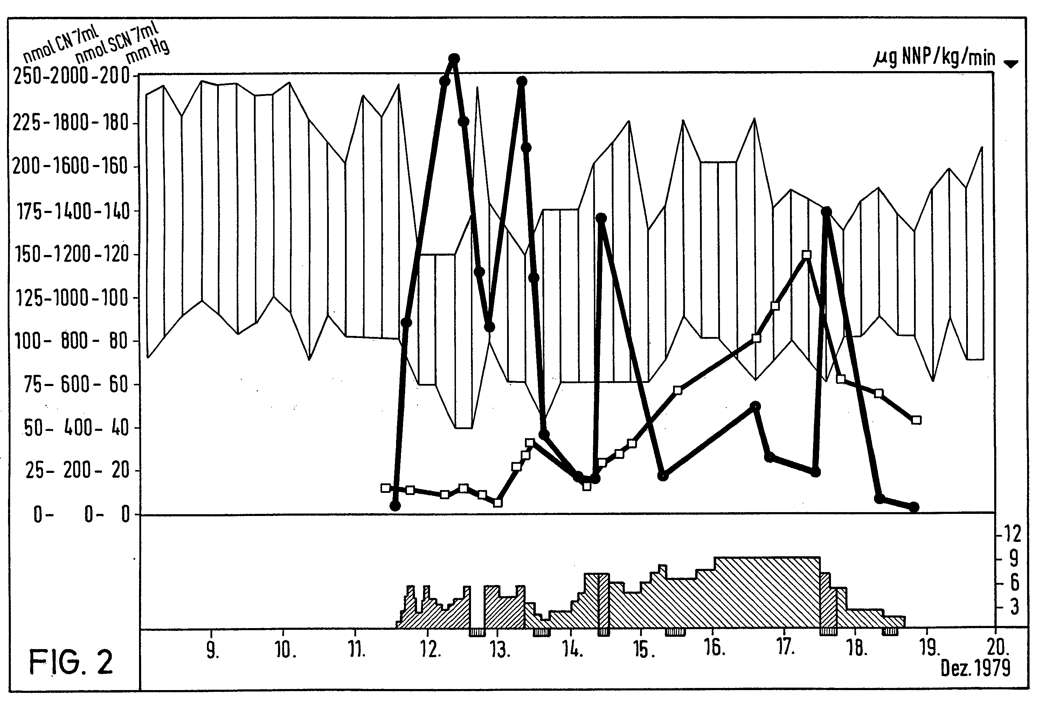

Abb. 1 und 2 zeigen den Wechsel der Monoinfusion von Natriumnitroprussid (hell schraffiert) und die kombinierte Infusion von Natriumnitroprussid mit Natriumthiosulfat (dunkel schraffiert). Wie ersichtlich, werden durch die zusätzlichen Infusionen von Natriumthiosulfat die bei der Therapie mit Natriumnitroprussid allein aufgetretenen hohen Cyanidblutspiegel gesenkt.

●—●: Zyanidspiegel im Blut

□—□: Thiozyanatspiegel im Blut.

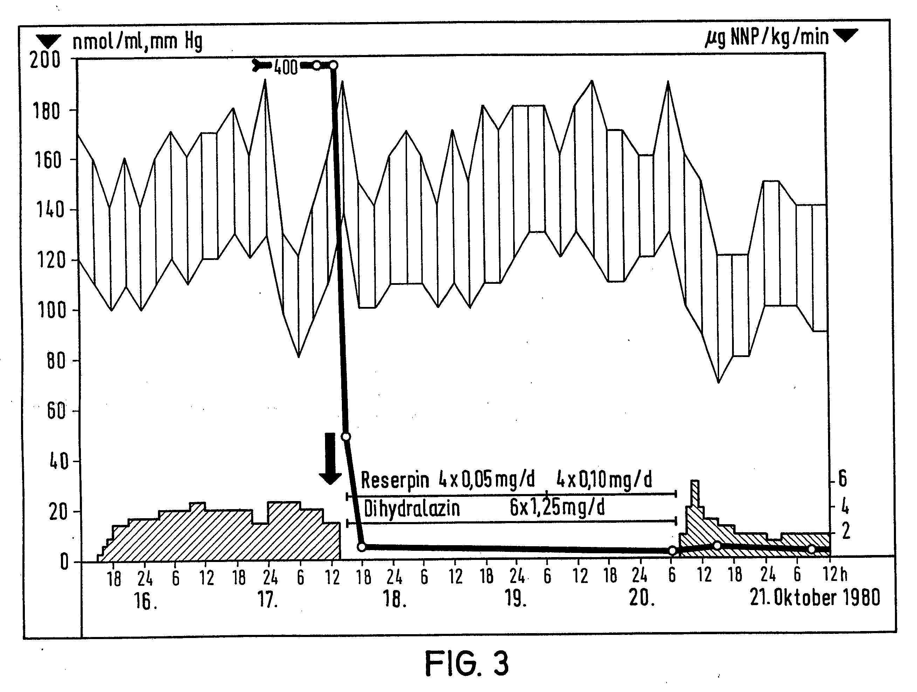

Abb. 3 zeigt den Wechsel von Monoinfusion von Natriumnitroprussid (hell-schraffiert) und Infusion der fertigen Mischlösung gem. der Erfindung (dunkel-schraffiert). Daraus wird ersichtlich, dass sich die bei der Therapie mit Natriumnitroprussid aufgetretenen hohen Cyanidblutspiegel durch die erfindungsgemässe Mischinfusion senken lassen

○—○: Cyanidspiegel im Blut.

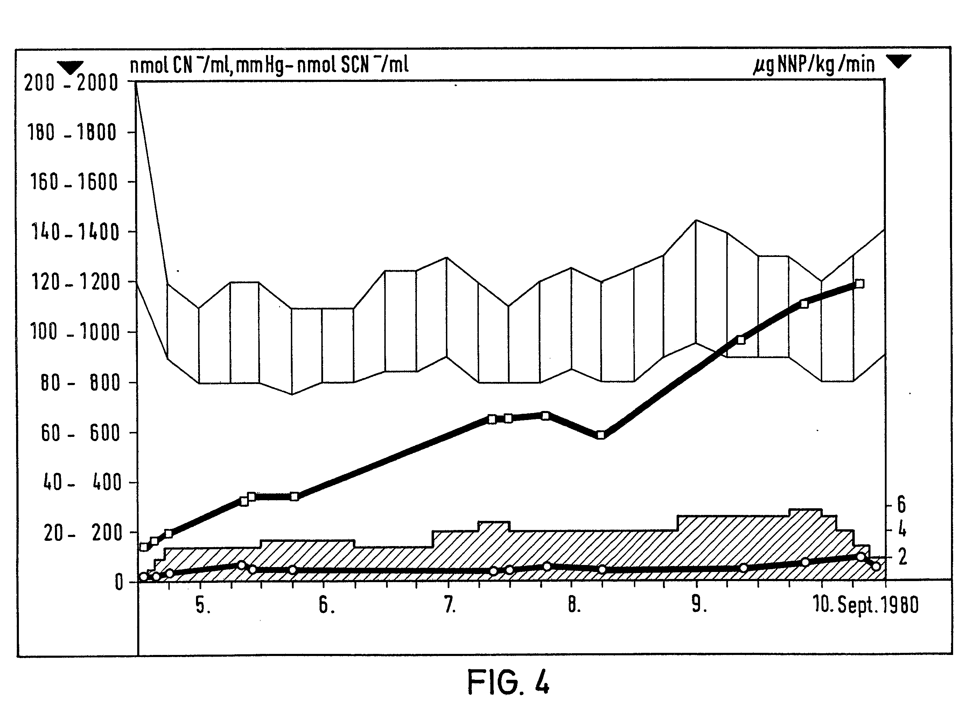

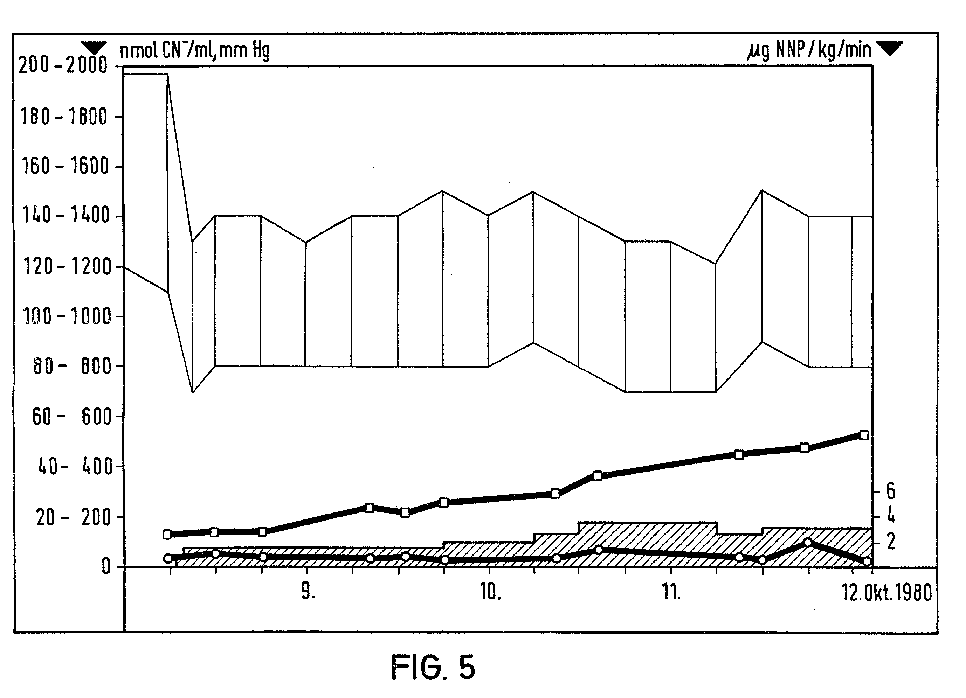

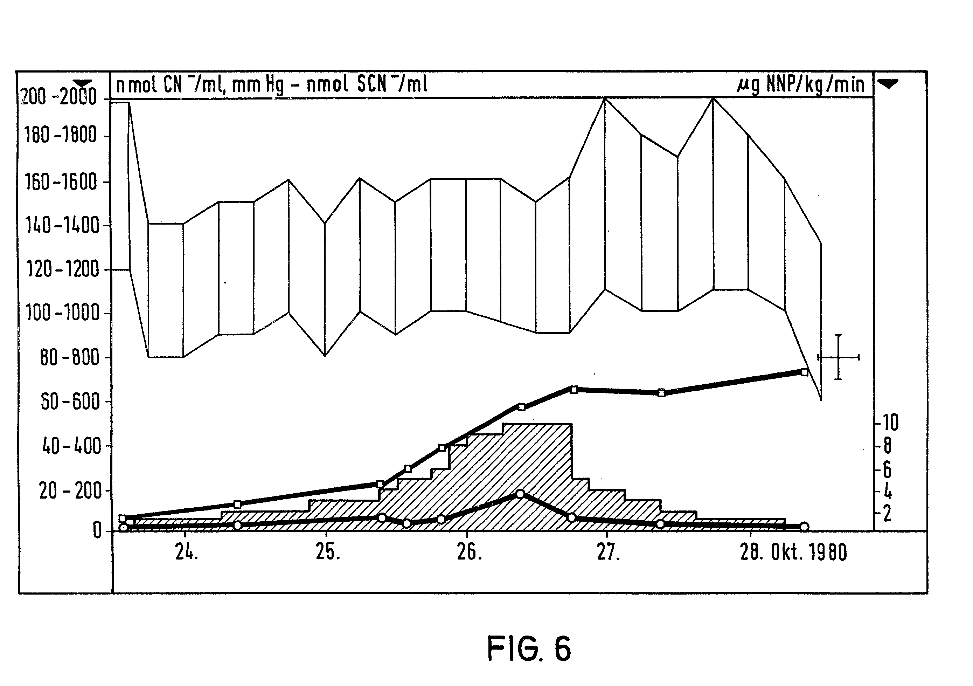

Abb. 4–6 zeigen die erfindungsgemässe Mischinfusion während des gesamten Therapiezeitraumes. Wie ersichtlich, findet von Anfang an kein Cyanidanstieg im Blut statt.

●—●: Zyanidspiegel im Blut,

□—□: Thiozyanatspiegel im Blut.

Bei allen Abbildungen 1–4 bedeutet die Doppelkurve mit Verbindungsstrichen im mittleren bis oberen Teil der Abbildungen den jeweils gemessenen Blutdruck.

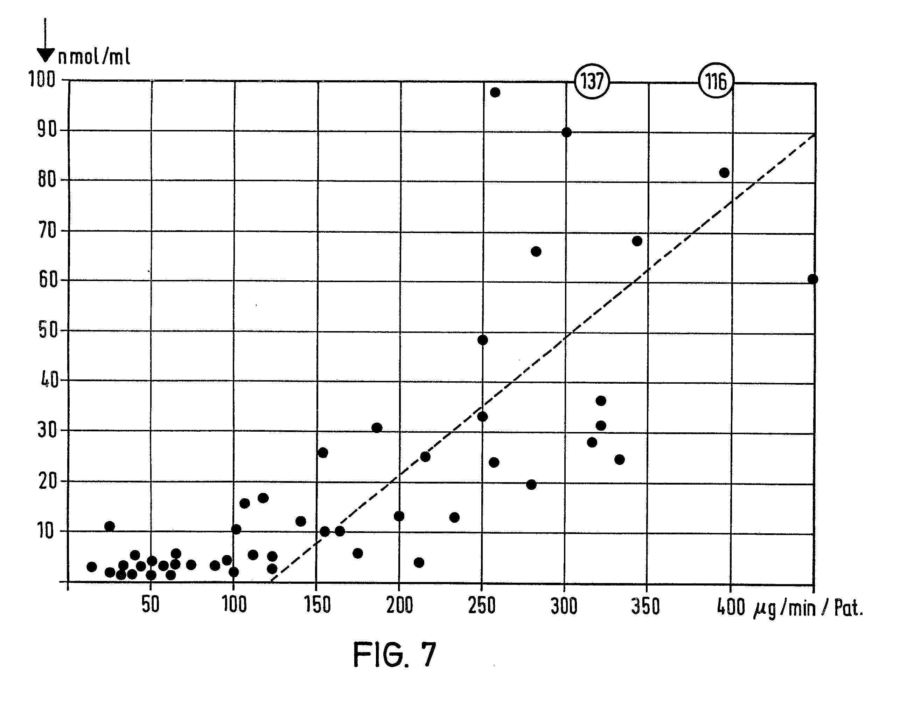

Abb. 7 zeigt die Cyanidmaximalkonzentrationen im Blut bei 3stündiger Monoinfusion von Natriumnitroprussid bei 50 Patienten in Abhängigkeit von den mittleren Dosisströmen. Die gestrichelte Linie bedeutet die rechnerisch zu erwartende Cyanidkumulation.

**Patentansprüche**

1. Natriumnitroprussid-Thiosulfat-Perfusor-Kit zur Bluthochdrucksenkung durch Infusion einer Mischlösung, enthaltend Natriumthiosulfat und Natriumnitroprussid, dadurch gekennzeichnet, dass er folgende Bestandteile enthält:

1 Flasche mit Durchstechstopfen enthaltend Natriumthiosulfatlösung;

1 Perfusorspritze aus lichtundurchlässigem Kunststoff mit beiliegender Kanüle zum Aufziehen der Lösung;

1 Perfusorschlauch aus lichtundurchlässigem Kunststoff;

1 Trockenampulle, enthaltend lyophilisiertes Natriumnitroprussid.

2. Kit nach Anspruch 1, dadurch gekennzeichnet, dass er folgende Bestandteile enthält:

1 Flasche mit Durchstechstopfen, enthaltend 50 ml 1%ige Natriumthiosulfatlösung;

1 50 ml-Perfusorspritze aus lichtundurchlässigem Kunststoff mit beiliegender Kanüle zum Aufziehen der Lösung;

1 Perfusorschlauch aus lichtundurchlässigem Kunststoff;

1 Trockenampulle, enthaltend 50 mg lyophilisiertes Natriumnitroprussid.

3. Natriumnitroprussid-Thiosulfat-Perfusor-Kit zur Bluthochdrucksenkung durch Infusion einer Mischlösung, enthaltend Natriumthiosulfat und Natriumnitroprussid, dadurch gekennzeichnet, dass er folgende Bestandteile enthält:

1 lichtundurchlässige Flasche mit Durchstechstopfen, enthaltend eine Mischlösung von Natriumthiosulfatlösung und darin gelöstem Natriumnitroprussid;

1 Perfusorspritze aus lichtundurchlässigem Kunststoff mit beiliegender Kanüle zum Aufziehen der Lösung;

1 Perfusorschlauch aus lichtundurchlässigem Kunststoff.

4. Kit nach Anspruch 3, dadurch gekennzeichnet, dass er folgende Bestandteile enthält:

1 lichtundurchlässige Flasche mit Durchstechstopfen, enthaltend eine Mischlösung von 50 ml 1%ige Natriumthiosulfatlösung und 50 mg darin gelöstem Natriumnitroprussid;

1 50 ml-Perfusorspritze aus lichtundurchlässigem Kunststoff mit beiliegender Kanüle zum Aufziehen der Lösung;

1 Perfusorschlauch aus lichtundurchlässigem Kunststoff.

## Claims

1. Sodium nitroprusside-thiosulfate perfusion kit for lowering blood hypertension by infusing a mixed solution containing sodium thiosulfate and sodium nitroprusside, characterized in that it contains the following components:

1 bottle with a piercable stopper containing sodium thiosulfate solution;

1 perfusion syringe made of a light-opaque synthetic material with a cannula closed by for aspirating the solution;

1 perfusion tubing made of a light-opaque synthetic material;

1 ampoule containing lyophilized sodium nitroprusside.

2. The kit according to claim 1, characterized in that it contains the following components:

1 bottle with a piercable stopper containing 50 ml of a 1% sodium thiosulfate solution;

1 50 ml perfusion syringe made of a light-opaque synthetic material with a cannula closed by for aspirating the solution;

1 perfusion tubing made of a light-opaque synthetic material;

1 ampoule containing 50 mg of lyophilized sodium nitroprusside.

3. Sodium nitroprusside-thiosulfate perfusion kit for lowering blood hypertension by infusing a mixed solution containing sodium thiosulfate and sodium nitroprusside, characterized in that it contains the following components:

1 light-opaque bottle with a piercable stopper containing a mixed solution comprising a sodium thiosulfate solution and sodium nitroprusside dissolved therein;

1 perfusion syringe made of a light-opaque synthetic material with a cannula closed by for aspirating the solution;

1 perfusion tubing made of a light-opaque synthetic material.

4. The kit according to claim 3, characterized in that it contains the following components:

1 light-opaque bottle with a piercable stopper containing a mixed solution comprising 50 ml of a 1% sodium thiosulfate solution and 50 mg of sodium nitroprusside dissolved therein;

1 50 ml perfusion syringe made of a light-opaque synthetic material with a cannula closed by for aspirating the solution;

1 perfusion tubing made of a light-opaque synthetic material.

## Revendications

1. Un kit pour perfusion comprenant du nitroprussiate et du thiosulfate de sodium pour l'abaissement de l'hypertension par perfusion d'une solution mixte contenant du thiosulfate de sodium et du nitroprussiate de sodium, caractérisé en ce qu'il comporte les éléments suivants:

1 flacon avec bouchon perforable contenant une solution de thiosulfate de sodium;

1 seringue de perfusion en matière synthétique opaque accompagnée d'une canule pour l'aspiration de la solution;

1 tuyau souple de perfusion en matière synthétique opaque;

1 ampoule de produit sec contenant du nitroprussiate de sodium lyophilisé.

2. Kit selon la revendication 1, caractérisé en ce qu'il comporte les éléments suivants:

1 flacon avec bouchon perforable contenant 50 ml de solution de thiosulfate de sodium à 1%;

1 seringue de perfusion de 50 ml en matière synthétique opaque accompagnée d'une canule pour l'aspiration de la solution;

1 tuyau souple de perfusion en matière synthétique opaque;

1 ampoule de produit sec contenant 50 mg de nitroprussiate de sodium lyophilisé.

3. Un kit pour perfusion comprenant du nitroprussiate et du thiosulfate de sodium pour l'abaissement de l'hypertension par perfusion d'une solution mixte contenant du thiosulfate de sodium et du nitroprussiate de sodium, caractérisé en ce qu'il comporte les éléments suivants:

1 flacon opaque avec bouchon perforable contenant une solution mixte d'une solution de thiosulfate de sodium et de nitroprussiate de sodium dissous dans celle-ci;

1 seringue de perfusion en matière synthétique opaque accompagnée d'une canule pour l'aspiration de la solution;

1 tuyau souple pour perfusion en matière synthétique opaque.

4. Kit selon la revendication 3, caractérisé en ce qu'il comprend les éléments suivants:

1 flacon opaque avec bouchon perforable contenant une solution mixte de 50 ml d'une solution de thiosulfate de sodium à 1% et de 50 mg de nitroprussiate de sodium dissous dans celle-ci;

1 seringue de perfusion de 50 ml en matière synthétique opaque accompagnée d'une canule pour l'aspiration de la solution;

1 tuyau souple pour perfusion en matière synthétique opaque.

FIG.1

FIG. 2

FIG. 3

FIG. 4

FIG. 5

FIG. 6

FIG. 7